**Europäisches Patentamt**

**European Patent Office**

**Office européen des brevets**

(19)

(11) Veröffentlichungsnummer: **0 045 913**
**B1**

(12) **EUROPÄISCHE PATENTSCHRIFT**

(45) Veröffentlichungstag der Patentschrift:
**20.11.86**

(21) Anmeldenummer: **81106032.6**

(22) Anmeldetag: **31.07.81**

(51) Int. Cl.⁴: **A 61 B 5/10**

(54) **Fingerabdrucksensor zum Erzeugen eines dem topografischen Relief eines zu untersuchenden Fingers entsprechenden elektrischen Signals.**

(30) Priorität: **11.08.80 US 176695**

(43) Veröffentlichungstag der Anmeldung:
**17.02.82 Patentblatt 82/7**

(45) Bekanntmachung des Hinweises auf die Patenterteilung:
**20.11.86 Patentblatt 86/47**

(84) Benannte Vertragsstaaten:
**AT BE CH DE FR GB IT LI NL SE**

(56) Entgegenhaltungen:
**FR - A - 2 407 530**
**US - A - 3 138 059**
**US - A - 3 882 462**
**US - A - 3 982 836**
**US - A - 4 120 585**

(73) Patentinhaber: **Siemens Aktiengesellschaft Berlin und München, Wittelsbacherplatz 2, D-8000 München 2 (DE)**

(72) Erfinder: **Rüll, Hartwig, Dr., Balduin-Helm-Strasse 39, D-8080 Fürstenfeldbruck (DE)**
Erfinder: **Devinney, Edward J., 100 Union Avenue, Delanco New Jersey 08075 (US)**
Erfinder: **Chiu, Ming-Yee, 78 Boothby Drive, Mt. Laurel, New Jersey 08054 (US)**

**Beschreibung**

Die Erfindung bezieht sich auf einen Fingerabdrucksensor zum Erzeugen eines dem topografischen Relief eines zu untersuchenden Fingers entsprechenden Signals.

Systeme zur Identifizierung von Fingerabdrükken, die den Abdruck eines auf eine Kontaktfläche gepressten Fingers identifizieren, sind bekannt.

Beispielsweise geht aus der US-PS 4 053 228 ein Apparat zur Identifizierung eines Fingers hervor, der eine transparente Glasplatte aufweist, die als eine Kontaktfläche oder als Fingerabdruckleser dient. Ein Fingerabdruck wird durch Andrükken des zu untersuchenden Fingers an die Rückfläche der Glasplatte gebildet, wobei der Finger in einer vorbestimmten Position gehalten wird. Der Fingerabdruck wird durch einen die Vorderfläche der Glasplatte durchstrahlenden Lichtstrahl abgefragt. Der abfragende Strahl wird an der Rückfläche teilweise reflektiert, so dass ein Signalstrahl entsteht, der die Fingerabdruckinformation trägt. Zur Identifizierung der Person wird der reflektierte Signalstahl dann mit einem Hologramm des gleichen Fingerabdrucks korreliert.

Aus der US-PS 4 120 585 geht ein anderes System zur Identifizierung eines Fingerabdrucks hervor. Dieses System enthält ein nachgiebiges optisches Prisma als Fingerabdruckleser. Die Basis des Prismas wird von dem Finger der zu untersuchenden Person berührt. Das elastische Prisma verformt sich unter dem ausgeübten Druck. Es reflektiert teilweise einen abtastenden Lichtstrahl in Richtung einer fotoempfindlichen Einrichtung, welche aktiviert wird. Die fotoempfindliche Einrichtung aktiviert ihrerseits weitere optische Komponenten des Identifizierungssystems. Der Fingerabdruckleser wird auf das aus Erhöhungen und Vertiefungen bestehende Muster des Fingerabdrucks einer zu identifizierenden Person hin überprüft.

Aus der US-A-3 138 059 ist ein Fingerabdrucksensor bekannt, bei dem eine auf einer Seite eines transparenten Körpers ausgebildete Kontaktfläche zum Aufdrücken des Fingers vorhanden ist. Der aufgedrückte Finger ist mit einem der Kontaktfläche durch den transparenten Körper hindurch zugeführten Lichtstrahl beleuchtbar, der von dem Fingerabdruck reflektiert wird, wobei der reflektierte Lichtstrahl dem beleuchteten Fingerabdruck entsprechend moduliert und einem lichtempfindlichen Feld zugeführt ist. Der durch den transparenten Körper der Kontaktfläche zugeführte Lichtstrahl ist dieser durch eine dazu im Winkel von 45° angeordnete teilreflektierende Fläche eines Strahlteilers so zugeführt, dass er senkrecht auf die Kontaktfläche trifft und den Fingerabdruck beleuchtet, wobei der von dem beleuchteten Fingerabdruck reflektierte Lichtstrahl durch Reflexion an der teilreflektierenden Fläche dem Feld lichtempfindlichen Feld zugeführt ist. Die Kontaktfläche kann eine Glasoberfläche, also eine inelastische Fläche sein, und das lichtempfindliche Feld ist ein fotografischer Film oder eine Streuplatte.

Ein System zur Identifizierung eines Fingerabdrucks sollte leicht und billig zusammengesetzt sein und gleichzeitig sollte es eine hohe Bildqualität des Fingerabdrucks liefern. Insbesondere sollte das Bild des Fingerabdrucks frei von Verzerrungen oder Verformungen sein.

Es ist daher die Aufgabe der Erfindung, einen Fingerabdrucksensor zu schaffen, der leicht zusammensetzbar ist, nur geringe Kosten erfordert und eine verbesserte Qualität der Fingerabdruckbilder liefert, die in einem weiten Bereich frei von Verzerrungen sind.

Diese Aufgabe wird durch die im kennzeichnenden Teil des Patentanspruchs 1 angegebene Merkmalskombination gelöst.

Durch diese Lösung ist ein Fingerabdrucksensor geschaffen worden, der die Fingerabdruckinformation eines berührenden Fingers in ein lesbares optisches Bild und in ein elektrisches Ausgangssignal umwandelt. Das elektrische Ausgangssignal des Fingerabdrucksensors repräsentiert die in dem Fingerabdruck enthaltene Information und kann zur Weiterverarbeitung in einen Rechner eingelesen werden.

Ein erfindungsgemässer Fingerabdrucksensor weist eine hohe Empfindlichkeit und Auflösung auf und arbeitet sehr zuverlässig.

Bevorzugte und vorteilhafte Ausführungsformen der Erfindung gehen aus den Unteransprüchen 2 bis 11 hervor.

Gemäss Anspruch 8 oder 9 wird bei einer bevorzugten Ausführungsform eines Fingerabdrucksensors zur Verbesserung des Kontrastes des Fingerabdruckbildes eine Schlierenmethode angewandt.

Eine bevorzugte Ausführungsform eines Fingerabdrucksensors, insbesondere gemäss Anspruch 10, erzeugt ein Ausgangssignal, welches eine digitale Verarbeitung des Fingerabdruckbildes ermöglicht.

Insbesondere ist bei einem erfindungsgemässen Fingerabdrucksensor keine Laserabtasttechnik erforderlich.

Zusammenfassend kann gesagt werden, dass durch die vorliegende Erfindung ein Fingerabdrucksensor zur Umwandlung der Fingerabdruckinformation eines berührenden Fingers in ein elektrisches Ausgangssignal geschaffen worden ist, der einen aus einem transparenten, elastischen Material gebildeten Kontaktkörper aufweist. Dieser Kontaktkörper enthält eine Kontaktfläche zur Aufnahme eines von dem zu untersuchenden Finger ausgeübten Kontaktdruckes. Wenn der Finger gegen die Kontaktfläche gedrückt wird, ändert sich die Struktur der Fläche entsprechend dem Fingerabdruckmuster, und zwar aufgrund der elastischen Eigenschaften des ausgewählten Materials. Nachdem der Finger von der Kontaktfläche weggenommen worden ist, nimmt diese ihre frühere Struktur wieder ein, d.h. sie wird wieder eben und glatt.

Der Fingerabdrucksensor weist ausserdem einen optischen Strahlteiler, eine Lichtquelle und ein erstes optisches System auf. Das erste optische System lenkt einen ersten Lichtstrahl aus der Lichtquelle über den optischen Strahlteiler und das transparente elastische Material zur Kontaktfläche des Kontaktkörpers. In Gegenwart eines andrückenden Fingers reflektiert oder sendet die Kontaktfläche einen Lichtstrahl zurück, der entsprechend dem Fingerabdruckmuster des Fingers moduliert ist.

Der Fingerabdrucksensor weist des weiteren ein zweites optisches System und einen Fotodetektor auf. Der Fotodetektor weist ein lichtempfindliches Feld zum Messen der Verteilung von Licht aus, das auf dieses Feld trifft. Das zweite optische System übermittelt das reflektierte und modulierte Licht von der Kontaktfläche über den Strahlteiler zum lichtempfindlichen Feld des Fotodetektors. Im zweiten optischen System ist ein Raumfilter enthalten, welches zum Blockieren eines Teils des reflektierten und modulierten Lichts vorgesehen ist (Schlierenmethode). Der Fingerabdrucksensor weist schliesslich einen Ausgang auf, der dem Fotodetektor zugeordnet ist und an dem das elektrische Ausgangssignal abnehmbar ist.

Das die Kontaktfläche des Kontaktkörpers bildende elastische Material kann aus einem Polymer bestehen. Insbesondere hat sich Silikonkautschuk oder -gummi, ein Elastomer, als brauchbar erwiesen.

Der Strahlteiler kann in Form eines Parallelepipeds ausgebildet sein, welches eine diagonale Fläche zum Reflektieren eines gewissen Anteils des einfallenden Lichts aufweist. Diese diagonale Fläche wird zum Trennen des von der Kontaktfläche reflektierten und modulierten Lichtstrahls von dem ersten Lichtstrahl benutzt. Vorzugsweise ist der Strahlteiler in Form eines Kubus ausgebildet. Ein kubischer Strahlteiler weist den Vorteil auf, dass Störungen oder Verzerrungen des Bildes minimal gehalten werden können.

Es ist möglich, einen optischen Strahlteiler zu benutzen, welcher ein transparentes elastisches Material enthält, und welcher wenigstens eine Fläche aufweist, die wenigstens so gross ist, wie der zu untersuchende Fingerabdruck. Diese Fläche kann als Kontaktfläche benutzt werden. In dieser Ausführungsform wird der optische Strahlteiler zugleich als Kontaktkörper benutzt.

Es ist auch möglich, für den Kontaktkörper und den optischen Strahlteiler zwei verschiedene Elemente zu benutzen. In dieser Ausführungsform kann eine Sensorplatte aus transparentem und elastischem Material vorgesehen sein. Diese Sensorplatte kann eine erste und zweite planare Seite aufweisen, welche parallel zueinander angeordnet sein können. Eine dieser Seiten kann als die Kontaktfläche zum Überprüfen eines auf sie ausgeübten Kontaktdruckes verwendet sein.

Bevorzugte Ausführungsformen der Erfindung sind in den Figuren dargestellt und werden in der folgenden Beschreibung näher erläutert. Von den Figuren zeigen

Fig. 1 einen Fingerabdrucksensor mit einem kubischen Strahlteiler und einer getrennten Kontaktplatte,

Fig. 2 eine Seitenansicht eines kubischen Strahlteilers, dessen Oberseite als Kontaktfläche zu benutzen ist,

Fig. 3 die Vorderansicht eines optischen Bandpassfilters, und

Fig. 4 die Filtercharakteristik des in Figur 3 dargestellten Bandpassfilters.

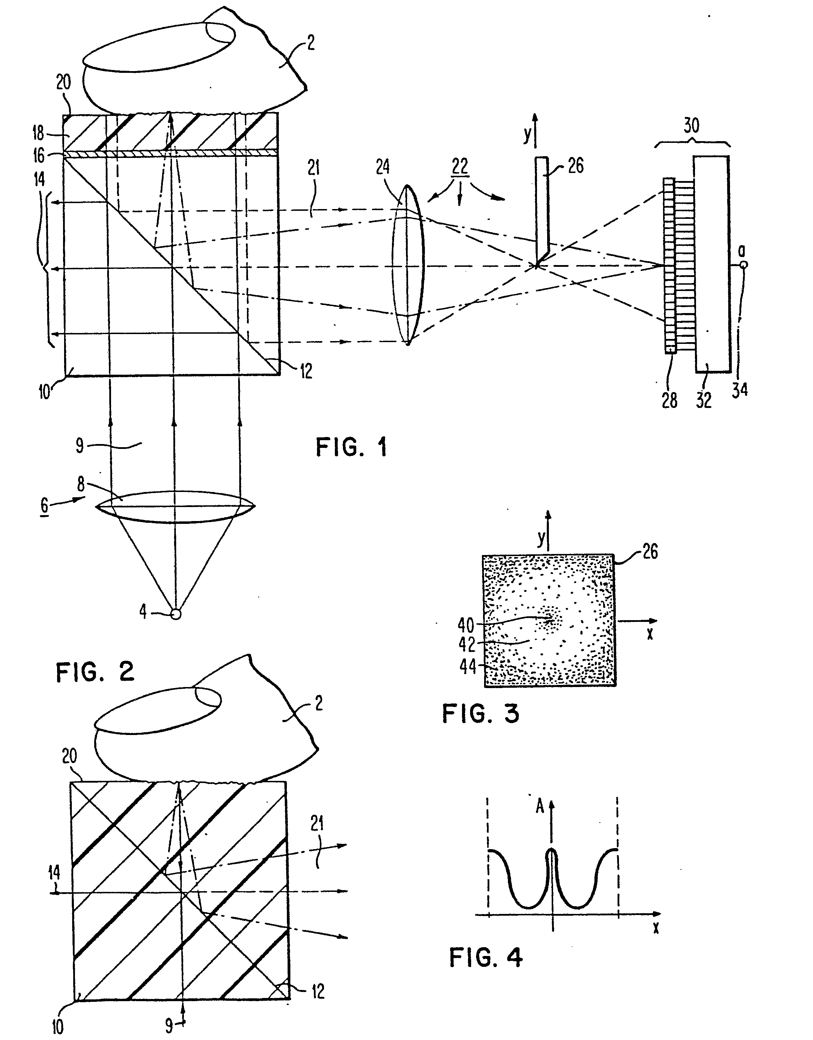

In der Figur 1 ist ein Fingerabdrucksensor zur Erzeugung eines dem topografischen Relief oder Muster eines zu untersuchenden Fingers 2 entsprechenden elektrischen Ausgangssignals a dargestellt.

Der Fingerabdrucksensor umfasst eine Lichtquelle 4, welche einen Lichtstrahl zum Abtasten des Fingerabdrucks aussendet. Die Lichtquelle 4 kann eine Weisslichtquelle, beispielsweise eine gewöhnliche Glühlampe sein. Die Lichtquelle 4 kann auch aus einer lichtemittierenden Diode (LED) oder aus einem Halbleiterlaser bestehen, die oder der Licht einer bestimmten Wellenlänge aussendet. Es ist jedoch einer der Vorteile der vorliegenden Erfindung, dass eine Lichtquelle 4, die ein breites Spektrum aussendet, verwendet werden kann.

Das Licht der Lichtquelle 4 wird von einem ersten optischen System 6 gesammelt, das eine Kollimatorlinse 8 enthält. Die Kollimatorlinse 8 sendet einen parallelen Lichtstrahl 9 in Richtung eines optischen Strahlteilers 10. Der parallele Lichtstrahl 9 tritt über die Unterseite des Strahlteilers und senkrecht zu ihr in diesen ein.

Der Strahlteiler 10 ist in Form eines Parallelepipeds ausgebildet. Insbesondere ist der Strahlteiler 10 würfelförmig ausgebildet. Bevorzugterweise ist der Strahlteiler 10 aus Glas gefertigt. Er weist eine diagonale Fläche oder Zwischenfläche 12 auf, die Licht, das auf die Unterseite oder die Oberseite der Fläche 12 auftrifft, teilweise reflektiert.

Ein gewisser Anteil 14 des einfallenden parallelen Lichtstrahls 9 wird nach links reflektiert und geht für das Verfahren zur Identifizierung des Fingerabdrucks verloren. Ein anderer Anteil des einfallenden Lichtstrahls 9 durchstrahlt die teilreflektierende Zwischenfläche 12 und erreicht die Oberseite des Strahlteilers 10. Dort durchstrahlt es ein optisches Anpassungsmedium 16. Das Medium 16 besteht aus einer Substanz, die den Brechungsindex des Strahlteilers 10 an den Brechungsindex eines benachbarten Kontaktkörpers anpasst. Insbesondere besteht dieser Kontaktkörper aus einer Sensorplatte 18, welche aus transparentem, elastischem Material gefertigt ist. Dieses elastische Material kann ein Elastomer, beispielsweise ein leichtes oder dünnes Kunststoffpolymer, sein. Vorzugsweise besteht die Sensorplatte 18 aus Silikonkautschuk oder -gummi.

Die Sensorplatte 18 weist eine obere und eine untere planare Seite auf. Beide Seiten sind parallel zueinander. Die obere Seite bildet eine Kontaktfläche 20 zur Aufnahme des Berührungsdruckes durch einen Finger 2. Wenn der Finger 2 die Sensorplatte 18 nicht berührt, ist die Kontaktfläche 20 glatt. Wenn der Finger 2 gegen die Kontaktfläche 20 gedrückt wird, wird die Oberflächenstruktur geändert. Die Kontaktfläche 20 erhält ein Muster, welches mit dem topografischen Relief der Erhöhungen und Vertiefungen der Haut des Fingers übereinstimmt.

Das Licht aus der Lichtquelle 4 durchstrahlt das Anpassungsmedium 16 und die Sensorplatte 18 und trifft schliesslich auf die Kontaktfläche 20, die von der Haut des zu überprüfenden Fingers 2 berührt wird. Dort findet teilweise eine Spiegelung statt.

Das von der Kontaktfläche 20 zurückkommende Licht ist in Form von einigen gestrichelten und einigen strichpunktierten Linien angedeutet. Das reflektierte Licht 21 ist mit der topologischen Struktur des Fingers 2 räumlich moduliert. Der Lichtstrahl 21 trägt demnach die Information über den Fingerabdruck. Er wird von der diagonalen Fläche 12 des Strahlteilers 10 nach rechts reflektiert. Danach durchstrahlt der Lichtstrahl 21 ein zweites optisches System 22.

Das zweite optische System 22 enthält eine Abbildungslinse 24, deren Durchmesser etwa der Höhe des Strahlteilers 10 entspricht. Der Lichtstrahl 24 durchstrahlt die Abbildungslinse 24 und einige zur optischen Vorverarbeitung dienende Elemente, beispielsweise ein Raumfilter 26 und ein nicht dargestelltes Farbfilter. Schliesslich trifft der reflektierte Lichtstrahl 21 auf das lichtempfindliche Feld 28 eines Fotodetektors 30. Der Fotodetektor 30 misst die Verteilung des auf sein lichtempfindliches Feld 28 auftreffenden Lichts. Dieses Feld 28 kann aus einem Feld fotoempfindlicher Elemente bestehen. Die Signale dieser Elemente werden einer digitalen Bildverarbeitungseinrichtung 32 zugeführt.

Der Fotodetektor 30 ist in Bezug auf die Abbildungslinse 24 so angeordnet, dass das Bild des Fingerabdrucks auf das lichtempfindliche Feld 28 fokussiert ist. Das Raumfilter 26, das eine Schneide sein kann, ist so angeordnet, dass es den reflektierten Lichtstrahl 21 teilweise ausblendet oder abblockt. Insbesondere ist es so angeordnet, dass es das Bild der Lichtquelle 4 ausblendet. Deshalb ist es in der Brennebene der Abbildungslinse 24 angeordnet.

Von einem Ausgang 34 des Fotodetektors 30 wird das elektrische Ausgangssignal a abgegeben. Es sei darauf hingewiesen, dass ein Strahlteiler 10 in Form eines Würfels gegenüber anderen Formen gewisse Vorteile aufweist. Der Hauptvorteil liegt in der Symmetrie der Lichtwege, was zu verzerrungsfreien Bildern auf dem lichtempfindlichen Feld 28 führt. Ein anderer Vorteil liegt darin, dass Strahlteilerwürfel leicht erhältlich sind.

Wie vorstehend erwähnt, kann ein Silikonpolymer oder Silikonkautschuk als transparentes Übertragungsmaterial für die Sensorplatte 18 verwendet werden. Der Silikonkautschuk kann beispielsweise SILGUARD® sein. Als Anpassungsmedium 16 kann Silikonöl verwendet werden. Dieses Öl erzeugt einen guten mechanischen Kontakt und eine gute optische Trennschicht zwischen der Sensorplatte 18 und dem Strahlteiler 10 aus Glas. Bei Verwendung von Silikonöl oder einem anderen Material als Anpassungsmedium 16 können vielfach Reflexionen vermieden werden.

Anstelle eines Silikonöls kann auch ein optischer Kitt mit richtig gewähltem Brechungsindex verwendet werden. Auch ein optischer Kleber mit den gleichen Eigenschaften kann verwendet werden.

In einer nicht dargestellten anderen Ausführungsform bedeckt die Sensorplatte 18, beispielsweise aus Silikonkautschuk die Oberseite des Strahlteilers 10 direkt. Bei dieser Ausführungsform ist ein Anpassungsmedium 16 nicht erforderlich.

Wie in der Figur 1 angedeutet, umfasst der Fotodetektor 30 ein lichtempfindliches Feld 28 und einen Schaltkreis 32 zur Signalverarbeitung. Der Fotodetektor 30 kann beispielsweise eine Standard-Videokamera (Vidikonkamera) sein. Eine derartige Videokamera weist den Vorteil auf, dass sie an einen Mikrocomputer anschliessbar ist. Jedoch ist es auch möglich, als Fotodetektor eine im Handel erhältliche CCD-Matrix oder eine CID-Matrix zu verwenden. Eine derartige Ausführung könnte der Einfachheit und Zuverlässigkeit wegen gewählt werden. Sie würde auch die Kosten und die Grösse des Fingerabdrucksensors verringern.

In der Figur 2 ist eine andere Ausführungsform eines vorgeschlagenen Fingerabdrucksensors dargestellt. Bei dieser Ausführungsform ist der optische Strahlteiler 10 aus einem transparenten elastischen Material gefertigt. Die Oberseite des würfelförmigen Strahlteilers 10 ist zugleich die Kontaktfläche 20. Die Kontaktfläche 20 wird unter dem Druck des Fingers 2 und entsprechend dem Fingerabdruck deformiert. Bei dieser Ausführungsform kann der Strahlteiler 10 mit Ausnahme der diagonalen Fläche 12 natürlich ganz aus Silikonkautschuk oder einem ähnlichen Kunststoffmaterial und/oder deformierbaren Material gefertigt sein.

Der optische Vorverarbeitungsschritt, der durch die Einfügung des Raumfilters 26 dargestellt ist, ist dazu vorgesehen, die Bildqualität des Fingerabdrucks zu verbessern. Die Anordnung einer Foucault-Schneide einer Messerschneide oder einer Rasierklinge in der Brennebene der Abbildungslinse 24 verbessert den Kontrast des Fingerabdruckbildes. Die Anwendung einer Messerschneide ist Teil eines sogenannten Schlierenaufbaus. Diese Technik ist in der Literatur beschrieben (siehe OPTICS, Febr. 1979, S. 478–481).

Das Raumfilter 26 kann, wie früher erwähnt, eine Schneide, insbesondere eine Messerschneide sein. Es kann auch gemäss den Figuren 3 und 4 ein räumliches Bandpassfilter sein. Das Bandpassfilter ist in der Ebene angeordnet, in welcher das Licht der Lichtquelle fokussiert ist. Die Funktion des räumlichen Bandpassfilters liegt darin, gewisse ungewollte Raumfrequenzen zu unterdrücken und ein Band gewünschter Frequenzen durchzulassen. Durch ein derartiges räumliches Bandpassfilter kann wieder eine Erhöhung des Kontrastes im Bild des Fingerabdrucks zusammen mit einer hohen Auflösung erreicht werden. Beispielsweise kann eine Glättung der Ränder und Rippen des Bildes des Fingerabdrucks erhalten werden. Dies erleichtert die digitale Verarbeitung des Bildes des Fingerabdrucks in hohem Masse.

Gemäss Figur 3 weist das Raumfilter 26 einen zentralen dunklen Fleck 40 auf, der von einem Feld 42 umgeben ist, der viel weniger dunkel ist. Dieses Feld 42 bildet einen Ring. Die äussere Region 44 des Raumfilters 26 ist wieder dunkel.

In der Figur 4 ist die Absorption A des Filters über dem Abstand x vom Zentrum des Filters 26 dargestellt. Der zentrale dunkle Fleck, der einen Bereich hoher Lichtabsorption A darstellt, ist der Grösse des Bildes der Lichtquelle 4 angepasst, das in der Brennebene der Abbildungslinse 24 erscheint. Der Ring 42 mit der niedrigen Lichtabsorption und damit hohen Transmission wirkt auf die Auflösung des Fingerabdruckbildes ein, das in der Ebene des lichtempfindlichen Feldes 28 erhalten wird.

Das räumliche Bandpassfilter 26 ist so ausgelegt, dass auf der einen Seite die Bildauflösung zum Einfangen aller feinen Details des Fingerabdrucks hoch genug ist und dass auf der anderen Seite undeutliche oder verschwommene Grate oder Erhöhungen unterdrückt werden.

**Patentansprüche**

1. Fingerabdrucksensor zum Erzeugen eines dem Papillarmuster der Haut eines Fingers (2) entsprechenden elektrischen Signals, mit einer auf einer Seite eines transparenten Körpers (18) ausgebildeten elastischen Kontaktfläche (20) zum Aufdrücken des Fingers (2), in der das jeweils aufgedrückte Papillarmuster durch elastische Verformung ein entsprechendes topografisches Relief erzeugt, das mit einem der Kontaktfläche durch den transparenten Körper (18) hindurch zugeführten Lichtstrahl (9) beleuchtbar ist, der von dem beleuchteten Relief reflektiert wird, wobei der reflektierte Lichtstrahl dem beleuchteten Relief entsprechend moduliert ist und einem lichtempfindlichen Feld (28) einer optoelektrischen Detektoreinrichtung (30) zur Erfassung der Verteilung der auf das Feld (28) auftreffenden Lichtintensität zugeführt ist, die ein dem topografischen Relief entsprechendes elektrisches Signal (a) erzeugt, das zugleich dem aufgedrückten Papillarmuster entspricht, dadurch gekennzeichnet, dass der durch den transparenten Körper (18) der Kontaktfläche (20) zugeführte Lichtstrahl (9) der Kontaktfläche (20) durch eine dazu im Winkel von 45° angeordnete teilreflektierende Fläche (12) eines Strahlteilers (10) so zugeführt ist, dass er senkrecht auf die Kontaktfläche (20) trifft und das Relief beleuchtet, und dass in dem von dem beleuchteten Relief reflektierten und durch Reflexion an der teilreflektierenden Fläche (12) dem lichtempfindlichen Feld (28) zugeführten Lichtstrahl (21) eine Abbildungsoptik (24) zum reellen Abbilden des beleuchteten Reliefs auf dem lichtempfindlichen Feld (28) sowie eine Raumfrequenzfiltereinrichtung (26) angeordnet sind, welche die im Relief hauptsächlich vorkommenden Raumfrequenzen zum lichtempfindlichen Feld (28) durchlässt, übrige Raumfrequenzen dagegen weitgehend unterdrückt.

2. Fingerabdrucksensor nach Anspruch 1, dadurch gekennzeichnet, dass das elastische Material aus einem Polymer besteht.

3. Fingerabdrucksensor nach Anspruch 2, dadurch gekennzeichnet, dass das elastische Material aus einem Silikonkautschuk besteht.

4. Fingerabdrucksensor nach einem der vorhergehenden Ansprüche, dadurch gekennzeichnet, dass eine aus dem transparenten elastischen Material gefertigte Sensorplatte (18) vorgesehen ist, die eine erste und zweite planare Seite aufweist, welche Seiten parallel zueinander sind, und dass eine der beiden Seiten die Kontaktfläche (20) zur Ausübung eines Kontaktdruckes bildet.

5. Fingerabdrucksensor nach einem der vorhergehenden Ansprüche, dadurch gekennzeichnet, dass der Strahlteiler (10) in Form eines Kubus ausgebildet ist.

6. Fingerabdrucksensor nach einem der vorhergehenden Ansprüche, dadurch gekennzeichnet, dass der Strahlteiler (10) aus Glas gefertigt ist.

7. Fingerabdrucksensor nach Anspruch 4 oder nach Anspruch 4 und Anspruch 5 oder 6, dadurch gekennzeichnet, dass die Sensorplatte (18) an dem Strahlteiler (10) angebracht ist und dass zwischen der Sensorplatte (18) und dem Strahlteiler ein mit beiden in Kontakt stehendes optisches Anpassungsmedium (16) angeordnet ist.

8. Fingerabdrucksensor nach einem der vorhergehenden Ansprüche, dadurch gekennzeichnet, dass die Raumfrequenzfiltereinrichtung (26) aus einer Schneide besteht, die in der Brennebene der Abbildungsoptik (24) angeordnet ist.

9. Fingerabdrucksensor nach einem der Ansprüche 1 bis 7, dadurch gekennzeichnet, dass die Raumfrequenzfiltereinrichtung (26) aus einem optischen Bandpassfilter besteht, das ein Zentrum (40) mit höherer Lichtabsorption aufweist, das von einem Bereich (42) mit niedrigerer Lichtabsorption umgeben ist und das in der Brennebene der Abbildungsoptik (24) angeordnet ist.

10. Fingerabdrucksensor nach einem der vorhergehenden Ansprüche, dadurch gekennzeichnet, dass die Fotodetektoreinrichtung (30) eine CCD-Matrix aufweist.

11. Fingerabdrucksensor nach einem der vorhergehenden Ansprüche, dadurch gekennzeichnet, dass die Fotodetektoreinrichtung (30) eine Videokamera aufweist.

**Claims**

1. A fingerprint sensor delivering an electrical signal corresponding to the papillary pattern of the skin of a finger (2) to be examined, comprising an elastic contact surface (20) designed on one side of a transparent body (18) for impression by the finger (2), wherein the respectively impressed papillary pattern produces a corresponding topographic relief by elastic deformation, which relief can be illuminated by a light beam (9) supplied to the contact surface through the transparent body (18) and reflected from the illuminated relief, where the reflected light beam is modulated in accordance with the illuminated relief and supplied to a light-sensitive field (28) of an opto-electric detector device (30) for detecting the distribution of the light intensity incident upon the field (28), which device (30) produces an electrical signal (a) which corresponds to the topographic relief and simultaneously corresponds to the impressed papillary pattern, characterised in that the light beam (9) supplied to the contact surface (20) through the transparent body (18) is supplied to the contact surface (20) through a partially-reflective surface (12) of a beam-splitter (10), which is arranged at an angle of 45° to said contact surface, in such a manner that it is vertically incident upon the contact surface (20) and illuminates the relief, and that in the light beam (21) reflected from the illuminated relief and supplied to the light-sensitive field (28) by means of reflection on the partially-reflective surface there are arranged both a focussing optical system (24) for focussing a real image of the illuminated relief on the light-sensitive field (28) and a spatial frequency filter device (26) which allows the predominant spatial frequencies in the relief to pass through to the light sensitive field (28), but greatly suppresses residual spatial frequencies.

2. A fingerprint sensor as claimed in Claim 1, characterised in that the elastic material consists of a polymer.

3. A fingerprint sensor as claimed in Claim 2, characterised in that the elastic material consists of a silicon rubber.

4. A fingerprint sensor as claimed in one of the preceding Claims, characterised in that there is provided a sensor plate (18) produced from the transparent elastic material and possessing a first and a second planar side, which sides are mutually parallel, and that one of the two sides forms the contact surface (20) for exerting a contact pressure.

5. A fingerprint sensor as claimed in one of the preceding Claims, characterised in that the beam-splitter (10) is in the form of a cube.

6. A fingerprint sensor as claimed in one of the preceding Claims, characterised in that the beam-splitter (10) is made of glass.

7. A fingerprint sensor as claimed in Claim 4, or as claimed in Claim 4 and Claim 5 or 6, characterised in that the sensor plate (18) is fixed to the beam-splitter (10) and that an optical matching medium (16) is arranged between the sensor plate (18) and the beam-splitter to connect said plate and beam-splitter.

8. A fingerprint sensor as claimed in one of the preceding Claims, characterised in that the spatial frequency filter device (26) consists of a cutting edge which is arranged in the focal plane of the focussing optical system (24).

9. A fingerprint sensor as claimed in one of Claims 1 to 7, characterised in that the spatial frequency filter device (26) consists of an optical bandpass filter with a centre (40) having high light absorption surrounded by a region (42) having low light absorption and arranged in the focal plane of the focussing optical system (24).

10. A fingerprint sensor as claimed in one of the preceding Claims, characterised in that the photodetector device (30) has a CCD matrix.

11. A fingerprint sensor as claimed in one of the preceding Claims, characterised in that the photodetector device (30) has a video camera.

**Revendications**

1. Détecteur d'une empreinte digitale pour produire un signal électrique correspondant au modèle pappilaire de la peau d'un doigt (2), comportant une surface de contact élastique (20) réalisée sur un côté d'un corps transparent (18), pour y appliquer le doigt (2) et dans laquelle le modèle pappilaire qui y a été appliqué produit, par déformation élastique, un relief topographique correspondant qui est susceptible d'être éclairé, à travers le corps transparent (18), à l'aide d'un rayon lumineux amené à la surface de contact et réfléchi par le relief éclairé, le rayon lumineux réfléchi étant modulé en fonction du relief éclairé et étant envoyé à un champ photosensible (28) d'un dispositif de détection optoélectronique (30) pour saisir la distribution de l'intensité lumineuse incidente sur le champ (28), qui produit un signal électrique (a) qui correspond au relief topographique et qui correspond en même temps au modèle pappilaire appliqué, caractérisé par le fait que le rayon lumineux (9) amené à la surface de contact (20) à travers le corps transparent (18), est amené à la surface de contact (20) par une surface à réflexion partielle (12) d'un diviseur de rayon (10), qui est inclinée à 45° sur ladite surface de contact, de manière à incider perpendiculairement sur celle-ci, et que dans le rayon lumineux (21) qui est réfléchi par le relief éclairé et amené, par réflexion sur la surface à réflexion partielle, au champ photosensible (28), sont disposés une optique de reproduction (24) pour former une image réelle du relief éclairé, sur le champ photosensible (28), ainsi qu'un dispositif à filtre de fréquences spatiales (26) qui laisse passer vers le champ photosensible (28), les fréquences spatiales qui se présentent principalement dans le relief, mais, par contre, supprime très largement d'autres fréquences spatiales.

2. Détecteur d'une empreinte digitale selon la revendication 1, caractérisé par le fait que le matériau élastique est constitué par un polymère.

3. Détecteur d'une empreinte digitale selon la revendication 2, caractérisé par le fait que le matériau élastique est constitué par un caoutchouc au silicone.

4. Détecteur d'une empreinte digitale selon l'une des revendications précédentes, caractérisé par le fait qu'il est prévu une plaque-captrice (18) faite avec un matériau transparent et élastique, ladite plaque comportant un premier et un second côté plan, lesdits côtés étant parallèles entre eux, et que l'un des deux côtés constitue la surface de contact (20) pour y exercer une pression de contact.

5. Détecteur d'une empreinte digitale selon l'une des revendications précédentes, caractérisé par le fait que le diviseur de rayon (10) est réalisé sous la forme d'un cube.

6. Détecteur d'une empreinte digitale selon l'une des revendications précédentes, caractérisé par le fait que le diviseur de rayon (10) est fait avec du verre.

7. Détecteur d'une empreinte digitale selon la revendication 4 ou selon les revendications 4 et 5 ou 6, caractérisé par le fait que la plaque-captrice (18) est montée sur le diviseur de rayon (10), et qu'entre la plaque-captrice (18) et le diviseur de rayon est prévu un milieu optique d'adaptation (16) qui est en contact avec ladite plaque et ledit diviseur.

8. Détecteur d'une empreinte digitale selon l'une des revendications précédentes, caractérisé par le fait que le dispositif à filtre de fréquences spatiales (26) est constitué par une lame qui est disposée dans le plan focal de l'optique de reproduction (24).

9. Détecteur d'une empreinte digitale selon l'une des revendications 1 à 7, caractérisé par le fait que le dispositif à filtre de fréquences spatiales (26) est constitué par un filtre optique passe-bande qui comporte un centre (40) à forte absorption de la lumière, entouré d'une zone (42) de moindre absorption de la lumière, et qui est disposé dans le plan focal de l'optique de reproduction (24).

10. Détecteur d'une empreinte digitale selon l'une des revendications précédentes, caractérisé par le fait que le dispositif photodétecteur (30) comporte une matrice CCD.

11. Détecteur d'une empreinte digitale selon l'une des revendications précédentes, caractérisé par le fait que le dispositif photodétecteur (30) comporte une caméra vidéo.

FIG. 1

FIG. 2

FIG. 3

FIG. 4